Europäisches Patentamt

European Patent Office

Office européen des brevets

(11) Veröffentlichungsnummer: **0 261 530 B1**

# (12) EUROPÄISCHE PATENTSCHRIFT

(45) Veröffentlichungstag der Patentschrift: **31.07.91**

(51) Int. Cl.⁵: **A61M 1/36**, B04B 13/00

(21) Anmeldenummer: **87113361.7**

(22) Anmeldetag: **12.09.87**

(54) Verfahren und Vorrichtung zur Trennung der Bestandteile des einem Spender "in vivo" entnommenen Blutes.

(30) Priorität: **22.09.86 DE 3632176**

(43) Veröffentlichungstag der Anmeldung:
**30.03.88 Patentblatt 88/13**

(45) Bekanntmachung des Hinweises auf die
Patenterteilung:
**31.07.91 Patentblatt 91/31**

(84) Benannte Vertragsstaaten:
**AT CH DE ES FR GB IT LI NL**

(56) Entgegenhaltungen:
**EP-A- 0 014 093**
**FR-A- 2 406 449**
**US-A- 3 489 145**
**US-A- 3 957 197**
**US-A- 4 151 844**

(73) Patentinhaber: **Fresenius AG**
**Gluckensteinweg 5**
**W-6380 Bad Homburg v.d.H.(DE)**

(72) Erfinder: **Neumann, Hans-Jürgen, Dr.**
**Dipl.-Phys.**
**Dillinger Strasse 13**
**W-6690 St. Wendel(DE)**

(74) Vertreter: **Dr. Fuchs, Dr. Luderschmidt**
**Dipl.-Phys. Seids, Dr. Mehler Patentanwälte**
**Abraham-Lincoln-Strasse 7**
**W-6200 Wiesbaden(DE)**

## Beschreibung

Die Erfindung bezieht sich auf ein Verfahren zur Trennung der Bestandteile des einem Spender "in vivo" einstellbar entnommenen Blutes (Vollblutes), das einstellbar mit Antikoagulantien versetzt, einer die Trennung bewirkenden, in der Drehzahl einstellbaren Blutzentrifuge zugeführt wird, aus der die separierten Bestandteile (Blutfraktionen) einstellbar abgezogen und danach zum Teil gesammelt sowie zum Teil zum Spender rückgeführt werden, bei dem die Fließraten des Vollblutes und der Fraktionen gemessen und selbsttätig so eingestellt werden, daß eine optimale Trennung erfolgt.

Die Erfindung betrifft ferner eine Vorrichtung zur Durchführung des Verfahrens.

Bei Trennsystemen dieser Art kommt es darauf an, daß die Ströme des Vollblutes und der Blutfraktionen in einem richtigen Verhältnis zueinander stehen sowie die Fraktionen im hohen Maße nur den separierten Bestandteil aufweisen, d.h. die sogenannte Trenngrenze (die Grenze zwischen roten Blutkörperchen und den anderen Fraktionen) in der Blutzentrifuge sich an einer optimalen Stelle befindet.

Ein Trennsystem vorgenannter Art ist durch die US-PS 3 489 l45 bekanntgeworden und dort in allen Details erläutert.

Die im bekannten Fall vorgesehenen Steueranordnungen der die Fließraten einstellenden Vorrichtungen (die insbesondere Förderpumpen enthalten) und der Drehzahlregler der Zentrifuge erlauben eine getrennte Einstellung der Fließraten der zugehörigen geförderten Medien sowie eine Drehzahlverstellung der Zentrifuge, um den vorgenannten Bedingungen Rechnung tragen zu können.

Der Zentrifuge ist ein Pufferbehälter für das mit Antikoagulantien versehene Vollblut zugeordnet, der eine Füllstandsregelung aufweist, die die Zufuhr von Vollblut automatisch unterbricht, wenn sich eine vorgegebene Vollblutmenge im Pufferbehälter befindet.

Trotz dieser Teilautomatisierung (Füllstandsregelung) bleibt es im bekannten Fall mit Nachteil der Bedienungsperson überlassen, manuell die Steuer- bzw. Regelvorrichtungen zu betätigen, um eine optimale Einstellung zu gewährleisten und diese Einstellung auch über längere Zeiträume beizubehalten (Nachstellen). Die Bedienungsperson überwacht dabei die Trenngrenze visuell. Bei vorgegebener Drehzahl der Zentrifuge kann über eine Änderung der Fördergeschwindigkeit der einzelnen Blutfraktionen zueinander, d.h. durch Änderung der Drehzahlen der zugehörigen Pumpen, die Trenngrenze verschoben werden (Spalte II, Zeile 64 bis Spalte l2, Zeile 3 der US-PS).

Das System nach der weiterhin bekanntgewordenen US-PS 4 l5l 844 macht von letzterem Zusammenhang Gebrauch. Es ist eine photoelektrische Abtasteinrichtung vorgesehen, die die Lage der Trenngrenze optisch erfaßt und in ein elektrisches Signal umwandelt, das mit einem Sollwert verglichen wird; die Differenz wirkt über einen digitalen Regler auf ein Stellglied ein, das die Einstellung der Vorrichtungen zum Transport der Fraktionen, abhängig von Größe sowie Betrag und Richtung des Anstieges der optischen Dichte an der Trenngrenze, gegenläufig verändert.

Zu der Steuerung bzw. Regelung der anderen Vorrichtungen und der Zentrifuge selbst macht die vorgenannte US-PS keine Aussage.

Dieses bekannte Prinzip der automatischen Ausregelung der Trenngrenze als Regelgröße über eine photoelektrische Verhältnisregelung der Fließraten der abgezogenen Fraktionen hat den erheblichen Nachteil, daß es die anderen variablen Systemgrößen nicht in die selbsttätige Regelung mit einbezieht. Schwankungen in der Vollblutzufuhr sowie der Zentrifugendrehzahl lassen sich daher nur ungenügend ausregeln. Ferner sind die Systemeinstellmöglichkeiten (Variationsbreite der Einstellmöglichkeiten im System) erheblich beschränkt.

Durch die DE-OS 28 45 399 ist ein weiteres, das eingangs bezeichnete Trennsystem bekanntgeworden, von dem die Erfindung ausgeht.

Bei diesem System ist das aus der erstgenannten US-PS 3 489 l45 bekanntgewordene System praktisch automatisiert worden, derart, daß bei vorgegebener Drehzahl die Fließraten des Vollblutes und der Fraktionen abhängig von dem Ansprechen eines optischen Meßfühlers bei Vermischen der Fraktionen (Hämatokritüberwachung analog der an zweiter Stelle genannten US-PS) so in Bezug aufeinander eingestellt werden, daß eine optimale Trennung erfolgt. Bei dem bekannten Trennsystem wird die Zentrifuge auf einen vorgegebenen Drehzahlwert eingestellt, so daß bei einem beliebig eingestellten Vollblutfluß bestimmte Fraktionsvolumina anfallen, deren unvermischte Entnahme das selbsttätige Steuerungssystem zu steuern und zu überwachen hat. Die für eine unvermischte Trennung notwendigen Abzugsgeschwindigkeiten der Fraktionen müssen daher ermittelt werden, d.h. es ist notwendig, bei fester Drehzahl und vorgegebenem Vollblutfluß den Anteil der einzelnen Fraktionen zu ermitteln und die Entnahme entsprechend dieser Ermittlung einzustellen. Bei jeder Änderung des Vollblutflusses muß daher das bekannte System aufs Neue komplett die anfallenden Fraktionen ermitteln.

Im bekannten Fall entstehen daher lange Regelzeiten und es ist daher nicht möglich, den Vollblutfluß sehr schnell spender- oder blutdruckbezogen einzustellen oder gar während einer Separation zu ändern. Darüber hinaus kann es in ungünstigen Fällen sogar passieren, daß die Drehzahl der Zentrifuge für den gewählten Vollblutfluß so ungünstig gewählt wurde, daß einer der gewünschten Fraktionen erst gar nicht anfallen und somit auch nicht abgezogen werden kann.

Der Erfindung liegt die Aufgabe zugrunde, das eingangs bezeichnete Verfahren und die zugehörige Vorrichtung so zu verbessern, daß der Vollblutfluß sehr schnell geändert werden kann.

Die Lösung dieser Aufgabe gelingt gemäß der Erfindung hinsichtlich des Verfahrens dadurch, daß man bei einer Änderung des Vollblutflusses die Drehzahl der Zentrifuge in der Weise verändert, daß das Verhältnis der in der Zentrifuge pro Zeiteinheit separierten Volumenteile der Blutfraktionen zum Volumen des in der Zeiteinheit in die Zentrifuge hineingeförderten Vollblutes vor und nach einer Änderung des Vollblutflusses annähernd gleich ist.

Hinsichtlich der Vorrichtung zur Steuerung eines Systems zur Trennung der Bestandteile des einem Spender "in vivo" entnommenen Blutes (Vollblutes) entsprechend diesem Verfahren gelingt die Lösung vorgenannter Aufgabe ausgehend von einer Vorrichtung mit mindestens einer Blutzentrifuge, der Vorrichtungen zur Blutabnahme, zur Förderung von Antikoagulantien, des entnommenen Blutes in die Blutzentrifuge und der separierten Bestandteile (Blutfraktionen), die zum Teil gesammelt und zum Teil zum Spender rückgeführt werden, zugeordnet sind, mit Steuer-/Regelanordnungen zur Einstellung der Fließraten des Antikoagulant, des Vollblutes sowie der verschiedenen Blutfraktionen und der Drehzahl der Zentrifuge, und mit einem Sensor zur Erfassung der Fließrate des Vollblutes, dessen Signal auf eine Steuerstufe geschaltet ist, deren Ausgang mit den Steuer-/Regelanordnungen verbunden ist, dadurch, daß die Übertragungsfunktion der Steuerstufe so bestimmt ist, daß bei einer Änderung des Vollblutflusses die Drehzahl der Zentrifuge in der Weise veränderbar ist, daß das Verhältnis der in der Zentrifuge pro Zeiteinheit separierten Volumteile der Blutfraktionen zum Volumen des in der Zeiteinheit in die Zentrifuge hineingeförderten Vollblutes vor und nach einer Änderung des Vollblutflusses annähernd das gleiche Verhältnis aufweisen.

Bei dem Trennsystem nach der Erfindung wird bei einer Änderung des Vollblutflusses die Zentrifugendrehzahl im "richtigen Verhältnis" mitgeändert, wobei dieses "richtige Verhältnis" durch eine Übertragungsfunktion bestimmt ist, d.h. die Übertragungsfunktion steuert das Anfallen der einzelnen Fraktionen.

Die Übertragungsfunktion braucht keine analytisch vorbestimmte Funktion zu sein; es kann sich auch um eine empirisch ermittelte Funktion handeln.

Bei einem beliebigen Vollblutfluß wird daher durch die korrekte Einstellung der Drehzahl zu diesem Vollblutfluß das optimale Anfallen der Fraktionen vorgegeben, so daß die notwendigen Abzugsgeschwindigkeiten für alle Fraktionen nicht ermittelt werden müssen, sondern von vornherein feststehen. Dadurch kann mit Vorteil der Vollblutfluß sehr schnell spender- oder blutdruckabhängig eingestellt und sogar während einer Separation geändert werden.

Fehleinstellungen der Separationsparameter derart, daß die Drehzahl der Zentrifuge für einen gewählten Vollblutfluß so ungünstig ist, daß eine der gewünschten Fraktionen erst gar nicht anfällt, sind bei der Erfindung von vornherein ausgeschlossen.

Darüber hinaus entfällt bei dem System nach der Erfindung die Detektion der während der Separation angefallenen Blutbestandteile, wodurch das System vereinfacht wird.

Anhand von in den Zeichnungen dargestellten Ausführungsbeispielen werden die Erfindung und die sie ausgestaltenden Merkmale näher beschrieben.

Es zeigen:

Fig. 1    eine Übersichtsdarstellung eines Blutzentrifugensystems mit der erfindungsgemäßen Steuerung,

Fig. 2    eine Übersichtsdarstellung des prinzipiellen Aufbaues einer Parameterkopplung zwischen Vollblutfluß und den Fließraten, des Antikoagulants, der Blutfraktionen und der Zentrifugendrehzahl,

Fig. 3    in einer schematischen Darstellung den Prinzipaufbau einer durch einen Mikroprozessor gebildeten digitalen Steuerstufe.

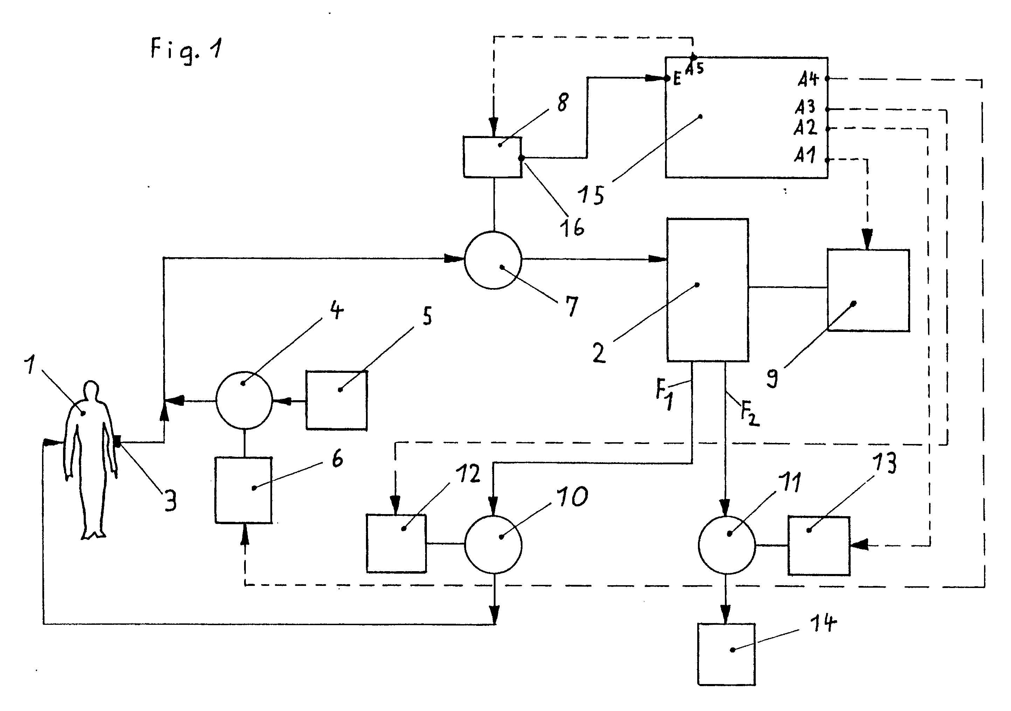

Die Figur 1 zeigt schematisch in Form einer Übersichtszeichnung ein System zur Trennung der Bestandteile des einem Spender 1 "in vivo" entnommenen Blutes in einer Blutzentrifuge 2, einschließlich der zugehörigen, erfindungsgemäß ausgebildeten Steuerung 15. Das System besitzt eine nicht näher dargestellte übliche Vorrichtung 3 zur Blutabnahme am Spender 1, eine Pumpe 4 zur Zuführung von Antikoagulantien, die in einem Behälter 5 bevorratet sind, zu dem entnommenen Blut. Der Pumpe 4 ist eine Steuer-/Regelanordnung 6 zur Einstellung der Fließrate des Antikoagulant zugeordnet.

Das System weist weiterhin eine Pumpe 7, die Blutpumpe, zur Förderung des entnommenen Vollblutes in die Blutzentrifuge 2 auf. Der Blutpumpe 7 ist eine Steuer-/Regelanordnung 8 zur Einstellung der Fließrate des Vollblutes zugeordnet.

Der Antrieb der Zentrifuge 2 weist eine Steuer-/Regelanordnung 9 zur Einstellung der Drehzahl der Zentrifuge auf.

An der Zentrifuge sind zwei Ausgänge zur Abnahme der separierten Blutbestandteile, der Blutfraktionen, nämlich der Plasmafraktion $F_1$ und der Zellenfraktion $F_2$ vorgesehen. Jedem Abnahmestrang ist eine Pumpe l0 bzw. ll zugeordnet. Die Fließraten der Blutfraktionen können durch den Pumpen jeweils zugeordnete Steuer-/Regelanordnungen l2, l3 eingestellt werden. Ein Teil der Blutfraktionen - hier die Zellenfraktion $F_2$ - wird in einem Behälter l4 gesammelt, wogegen der andere Teil der Blutfraktionen, die Plasmafraktion, zum Spender zurückgeführt wird. Es ist dabei auch bekannt, die Zellenfraktion zum Teil wieder dem entnommenen Blut rückzuführen.

Vorstehend beschriebenes System ist durch die eingangs genannte US-PS bekannt und dort in allen Einzelheiten beschrieben.

Um eine selbsttätige optimale Steuerung des Systems zu gewährleisten, ist eine Steuerung l5 vorgesehen, die als Eingangssignal - direkt oder mittelbar - ein Signal über die Fließrate des Vollblutes erhält, das von einem Sensor l6 erzeugt wird. Der Ausgang der Steuerung wirkt mit einem ersten Signal (Ausgang A l) auf die Steuer-/Regelanordnung 9 zur Verstellung der Drehzahl der Zentrifuge ein. Die Ausgänge A 2 bis A 3 sind mit den Steuer-/Regelanordnungen l2, l3 der Fördervorrichtung l0, ll der Blutfraktionen $F_1$ und $F_2$ verbunden. Der Ausgang A 4 steht mit der Steuer-/Regelanordnung 6 der Fördervorrichtung 4 des Antikoagulant in Verbindung. Der Ausgang A 5 ist auf die Einstellanordnung 8 für das Vollblut zurückgeführt.

Die Übertragungsfunktion der Steuerung l5 ist hinsichtlich des Ausganges A l erfindungsgemäß so bestimmt, daß bei einer Änderung des Vollblutflusses die Drehzahl der Zentrifuge in der Weise veränderbar ist, daß das Verhältnis der in der Zentrifuge pro Zeiteinheit separierten Volumteile der Blutfraktionen zum Volumen des in der Zeiteinheit in die Zentrifuge hineingeförderten Vollblutes vor und nach einer Änderung des Vollblutes annähernd das gleiche Verhältnis aufweisen, oder mathematisch ausgedrückt:

$$\frac{\text{Volumen}(\text{Fraktion}_n)_t}{\text{Volumen}(\text{Vollblut})_t} = \frac{\text{Volumen}(\text{Fraktion}_n)_{t+\Delta t}}{\text{Volumen}(\text{Vollblut})_{t+\Delta t}}.$$

Um dies zu gewährleisten, ist nach einer Ausgestaltung der Erfindung die Übertragungsfunktion mit Vorteil so bestimmt, daß bei einer Änderung des Vollblutflusses und des zugehörigen Eingangssignales am Eingang der Steuerstufe das Ausgangssignal (Al) zur Einstellung der Drehzahl der Zentrifuge proportional der Quadratwurzel aus dem neu eingestellten Vollblutfluß ist, d.h.

$$(1) \quad \text{Drehzahl}\ (\text{Zentrifuge}) = C_5 \times \sqrt{\text{Fluß}\ (\text{Vollblut})}.$$

Diese Übertragungsfunktion gewährleistet eine optimale Einstellung der Systemparameter abhängig von den Schwankungen im Vollblutfluß.

Hinsichtlich der Ausgänge A 2 - A 4 ist die Übertragungsfunktion der Steuerung l5 so bestimmt, daß bei einer Änderung des Vollblutflusses zusätzlich Ausgangssignale für die Einstellung der Fließraten von Antikoagulantien und aller Blutfraktionen erzeugbar sind, die proportional der Größe des Vollblutflusses sind, oder mathematisch ausgedrückt

$$(2) \quad \text{Fluß}\ (\text{Antikoagulant}) = C_4 \times \text{Fluß}\ (\text{Vollblut})$$

$$(3) \quad \text{Fluß}\ (\text{Fraktion}_n) = C_n \times \text{Fluß}\ (\text{Vollblut})$$

n > 2 = Index der Fraktion.

Dadurch ist eine umfassende, optimale und flexible Einstellung des Systems gewährleistet.

Die Proportionalitätsfaktoren $C_4$, $C_5$, $C_n$, die sogenannten Koppelkonstanten, sind vorgegeben und in der Steuerung in Form von digitalen oder analogen Größen dauerhaft abgelegt (gespeichert) und können so für den Betrieb den zugehörigen Steuer-/Regelanordnungen die gewünschten Proportionalitäten mitteilen.

Die Steuerung l5 ist so aufgebaut, daß die Fließrate des Antikoagulants oder der einzelnen Blutfraktionen oder die Drehzahl der Zentrifuge jeweils einzeln, entkoppelt von den anderen Größen, veränderbar sind, insb. ohne Rückwirkung auf den Vollblutfluß, indem die zugehörige Koppelkonstante geändert wird und der geänderte Wert in der Steuerstufe abgespeichert ist, d. h.

$$(4) \quad \text{Fluß (Antikoagulant)} \quad = C_4' \text{ x Fluß (Vollblut)}$$

$$(5) \quad \text{Fluß (Fraktion)} \quad = C_n' \text{ x Fluß (Vollblut)}$$

$$(6) \quad \text{Drehzahl (Zentrifuge)} \quad = C_5' \text{ x } \sqrt{\text{Fluß (Vollblut)}}.$$

Eine Änderung beispielsweise des Zellenflusses bildet sich dann als Änderung der zugehörigen Koppelkonstante ab.

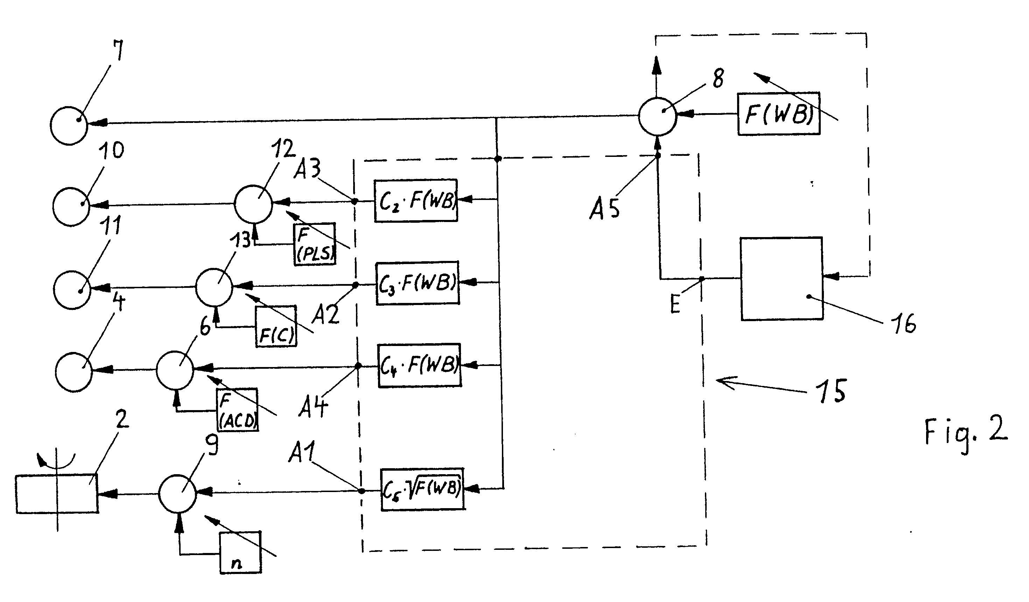

Diese Zusammenhänge sind in Figur 2 schematisch in steuerungstechnischer Ausdrucksweise dargestellt. Es bedeuten dabei
F (WB) Vollblutfluß
$C_2$ Koppelkonstante für den Plasmafluß F(PLS)
$C_3$ Koppelkonstante für den Zellenfluß F(C)
$C_4$ Koppelkonstante für den Antikoagulantfluß F(ACD)
$C_5$ Koppelkonstante für die Zentrifugendrehzahl n.

Im linken Teil der Figur 2 sind die Pumpen 7, 10, 11 und 4 dargestellt. Die Kästchen mit einem Diagonalpfeil sind die den Pumpen zugeordneten Steuer-/Regelanordnungen 6, 8, 9, 12, 13. In den anderen Kästchen werden die nachstehenden Produkte aus dem Vollblutfluß und den Koppelkonstanten gebildet, die die Größe der einzustellenden Flüsse und der Zentrifugendrehzahl, d.h. die Sollwerte für die Steuer-/Regelanordnungen der Pumpen und des Zentrifugenmotors darstellen:

$F(PLS) = C_2 \cdot F(WB)$ Plasmafluß
$F(C) = C_3 \cdot F(WB)$ Zellenfluß
$F(ACD) = C_4 \cdot F(WB)$ Antikoagulantfluß

$$n = C_5 \cdot \sqrt{F(WB)} \quad \text{Zentrifugendrehzahl.}$$

Die Führungsgröße für die Einstellung des Vollblutflusses selbst wird zweckmäßig einer Eingangsdrucksteuerung (mit dem "Sensor" 16) entnommen, die später noch erläutert wird.

Der Aufbau des Systems und der Steuerung kann mit konventionellen Bauelementen erfolgen. So sind zweckmäßig die Fördereinrichtungen 7 und 10, 11 sowie 4 für Vollblut und Blutfraktionen sowie für Antikoagulant peristaltisch arbeitende Schlauchpumpen, bei denen der Fluß mit einer Steuer- oder Regeleinrichtung für die Drehzahl der Pumpen eingestellt wird.

Die Vorrichtung 7 zur Förderung des Vollblutes ist zweckmäßig eine Pumpe (Blutpumpe), die auf konstanten Eingangsdruck regelbar ist, deren Drucksensor gleichzeitig der Sensor 16 für die Fließrate des Vollblutes ist. Derartige Pumpen verhindern, daß bei Abfall des Blutdruckes des Spenders und unverminderter Drehzahl (Abgaberate) der Blutabnahmeschlauch kolabiert. Die Blutpumpe weist in diesen Fällen ein unterlagertes Regelsystem (8) für eine Änderung der Fließrate des Vollblutes auf, das so aufgebaut ist, daß es die Fließrate des Vollblutes - und gekoppelt damit die anderen Systemgrößen - so einstellt, wie es dem am Eingang der Blutpumpe per Zeiteinheit angebotenen Blutvolumen entspricht.

Zweckmäßig ist dabei ein Drucksensor für den Eingangsdruck der Blutpumpe vorgesehen, der das Istwertsignal für das Regelsystem bildet.

Der Sollwert des Regelsystems kann ein vorbestimmter Druckwert sein, so daß das Regelsystem den Vollblutfluß der Blutpumpe so einstellt, daß der Eingangsdruck einen konstanten, dem Sollwert entsprechenden Wert annimmt. Dem Regelsystem kann auch ein vorbestimmter Sollwertbereich zugeordnet sein, so daß der Fluß der Blutpumpe so einstellbar ist, daß der Eingangsdruck in dem Sollwertbereich liegt.

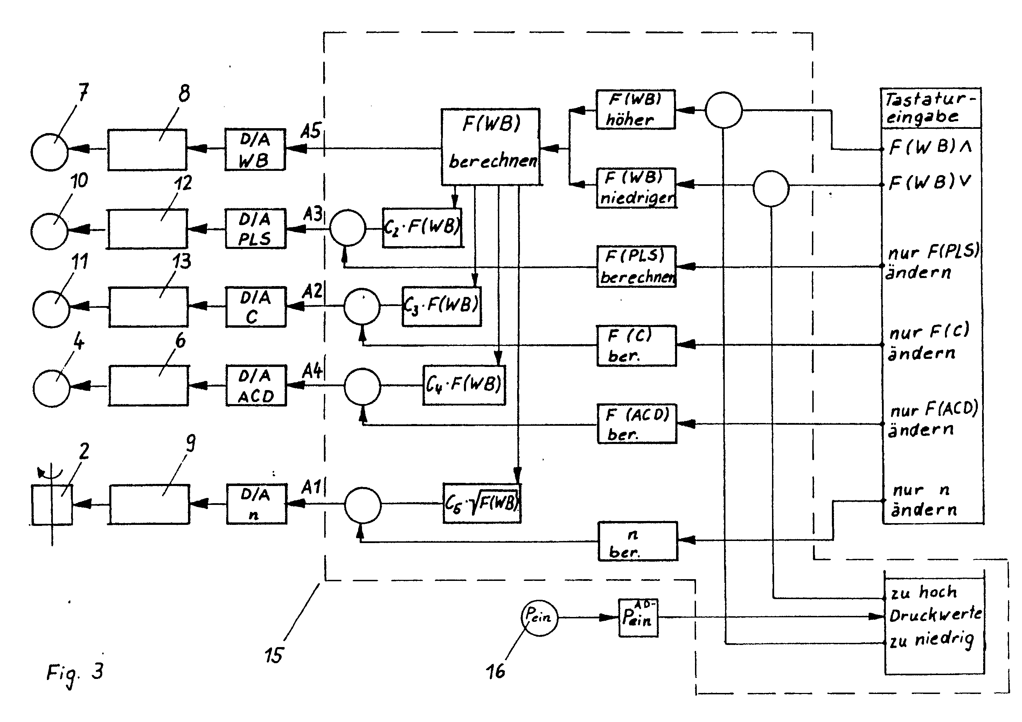

Die Steuerung kann prinzipiell aus fest verschalteten Rechengliedern aufgebaut werden. Vorteilhaft ist jedoch, die Steuerung 15 durch einen als digitalen Programmregler arbeitenden Mikroprozessor zu realisieren, der die Flexibilität und die Variationsbreite der Einstellmöglichkeiten erheblich erhöht. Ein derartiges System ist in Figur 3 schematisch dargestellt, das nunmehr in den Grundsätzen erläutert werden soll.

Nach dem Einschalten des Systems sind insbesondere die Werte über den Vollblutfluß F(WB) - Werte 1.1 - und die Koppelkonstanten $C_2$ bis $C_5$ in einem digitalen Speicher fest abgespeichert.

5

EP 0 261 530 B1

Im sogenannten Separationsprogrammteil werden folgende Betriebsparameter mit Hilfe dieser vorgegebenen, abgespeicherten Werte vom Mikroprozessor berechnet:

$$1.2 \quad \text{Plasmafluß F(PLS)} \quad = C_2 \cdot F(WB)$$
$$1.3 \quad \text{Zellenfluß F(C)} \quad = C_3 \cdot F(WB)$$
$$1.4 \quad \text{Antikoagulantfluß F(ACD)} \quad = C_4 \cdot F(WB)$$
$$1.5 \quad \text{Zentrifugendrehzahl n} \quad = C_5 \cdot \sqrt{F(WB)}.$$

Anschließend werden vom Mikroprozessor entsprechende Ausgaben über nachgeschaltete Digital-/Analogwandler auf die Steuer-/Regelanordnungen 12, 13, 6, 9 durchgeführt,so daß sich durch Verstellen der Pumpen 10, 11, 4 und des Motors der Zentrifuge 2 die entsprechenden Flüsse und die berechnete Drehzahl einstellen. Dieses Separationsprogramm entspricht der Darstellung der Figur 2.

Das Mikroprozessorsystem erlaubt - über das Separationsprogramm hinaus - einen Programmteil "Parameterkopplung an den Vollblutfluß" genannt.

Über eine Tastatureingabe für das Mikroprozessorsystem - obere Reihe in Figur 3, rechter Teil - hat man die Möglichkeit, den Vollblutfluß für das Separationsprogramm zu ändern:

2.1 $F(WB) \rightarrow F'(WB)$

und: $F(WB) = F'(WB)$ wird abgespeichert.

Es wird daher, wie in der oberen Zeile der Figur 3 dargestellt, jeweils der geänderte Vollblutfluß F(WB) berechnet, auf den Regler 8 der Blutpumpe geschaltet und als neuer Rechenwert F(WB) abgespeichert. Mit Hilfe der abgespeicherten Koppelkonstanten $C_2$, $C_3$, $C_4$, $C_5$ werden unmittelbar danach die übrigen Betriebsparameter mitgeändert:

2.2 $F(PLS) \rightarrow F'(PLS) = C_2 \cdot F(WB)$
und:
2.3 $F(C) \rightarrow F'(C) = C_3 \cdot F(WB)$
und:
2.4 $F(ACD) \rightarrow F'(ACD) = C_4 \cdot F(WB)$
und:

$$2.5 \quad n \longrightarrow n' \quad = C_5 \cdot \sqrt{F(WB)}$$

Derartige Änderungen des Vollblutflusses können vor während des Separationsprogrammes beliebig oft durchgeführt werden, wobei jeweils der letzte Parametersatz nach 2.1 bis 2.5 vom Mikroprozessor im Separationsprogramm ausgeführt wird.

Das Mikroprozessorsystem weist ferner einen Programmteil "Parameteränderung ohne Parameterkopplung" auf.

Über die Tastatureingabe (Reihen 2 bis 5) hat man die Möglichkeit, folgende Separationsparameter einzeln zu ändern, ohne daß dieses Einfluß auf einen anderen Betriebsparameter hat.

3.2 Änderung des Plasmaflusses

$F(PLS) \rightarrow F'(PLS) = C_2' \cdot F(WB)$

Es wird nach der Änderung $C_2 = C_2'$ als neue Koppelkonstante vom Mikroprozessorsystem neu abgespeichert!

3.3 oder Änderung des Zellenflusses:

$F(C) \rightarrow F'(C) = C_3' \cdot F(WB)$

und $C_3 = C_3'$ als neue Koppelkonstante

3.4 oder Änderung des Antikoagulantflusses

$F(ACD) \rightarrow F'(ACD) = C_4' \cdot F(WB)$

6

und $C_4 = C_4'$ als neue Koppelkonstante
3.5 oder Änderung der Zentrifugendrehzahl

$$n \longrightarrow n' = C_5' \cdot \sqrt{F(WB)}$$

und $C_5 = C_5'$ als neue Koppelkonstante

Änderungen der Betriebsparameter nach 3.2 bis 3.5 können beliebig oft und in beliebiger Kombination durchgeführt werden, wobei jeweils der letzte Parametersatz $C_2$, $C_3$, $C_4$, $C_5$ vom Mikroprozessorsystem ausgeführt wird.

Eine "Parameterkopplung an den Vollblutfluß" nach einer Parameteränderung gemäß einem der Schritte 3.2 bis 3.5 wird mit dem letzten abgespeicherten Parametersatz F(WB), $C_2$, $C_3$, $C_4$, $C_5$ nach den vorgenannten Schritten 2.2 bis 2.5 durchgeführt.

Die "Parameterkopplung an den Vollblutfluß" erfolgt zweckmäßig mit einer Drucksteuerung des Vollblutflusses, die in Figur 3 im rechten, unteren Teil der Zeichnung dargestellt ist und als optionale eingangsdruckgesteuerte Parameterkopplung bezeichnet wird. Es ist ein Drucksensor l6 vorgesehen, der den Eingangsdruck erfaßt und über einen Analog/Digitalwandler in den Mikroprozessor eingibt.

Es sind drei Varianten dieser Kopplung denkbar.

4.l Nur Unterdrucksteuerung (sogenannte high flow Überwachung).

Bei dieser Variante wird lediglich mittels einer Druckmessung überwacht, ob der eingestellte Blutfluß hoch ist.

Es werden dabei folgende Verfahrensschritte (Zyklus) durchgeführt:

4.l.l Nach dem Start des Separationsprogrammes laufen alle Pumpen und die Zentrifuge auf die voreingestellten Werte gemäß den vorher beschriebenen Schritten.

4.l.2 Der Eingangsdruck wird gemessen und von einem Mikroprozessorsystem oder einer sonstigen elektronischen Schaltung überwacht:

4.l.3 Der Eingangsdruck $P_{ein}$ sinkt unter eine zulässige Schwelle $P_{min}$, d.h. F(WB) ist zu hoch.

4.l.4 Es wird ein neuer Vollblutwert errechnet

$$F'(WB) = F(WB) - \Delta F(WB)$$

$\Delta F(WB)$ = Vollblutflußerniedrigung (ein Schritt z.B. 5 ml/min)

4.l.5 Danach erfolgt eine Parameterkopplung an den Wert F'(WB) nach den erläuterten Schritten 2.2 bis 2.5.

4.l.6 Eine maximale untere Grenze für F'(WB) sollte vorgegeben sein (z.B. F'(WB) > 25 ml/min), um bei eventuell hoher oder vollständiger Okklusion von Eingangsleitung oder Entnahmenadel den Wert F'(WB) = 0 nicht als Betriebsparameter zuzulassen.

4.l.7 Der nächste Zyklus beginnt dann wieder mit Schritt 4.l.2

4.2 Vollblutflußoptimierung nur in einer Anfangsphase

In einer Optimierungsphase am Anfang wird einmal der Vollblutfluß optimiert (d.h. maximiert); nach Abschluß dieser Phase wird lediglich überwacht, ob dieser Fluß für die Behandlungsdauer aufrechterhalten werden kann, oder ob sie erniedrigt werden sollen.

Bei dieser Variante werden nachstehende Schritte vollzogen

4.2.l Nach dem Start des Separationsprogrammes laufen alle Pumpen und die Zentrifuge auf die voreingestellten Werte gemäß den vorher beschriebenen Schritten.

4.2.2 Der Eingangsdruck wird gemessen und von einem Mikroprozessorsystem oder einer sonstigen elektronischen Schaltung überwacht.

4.2.3 $P_{ein}$ liegt oberhalb einer bestimmten Schwelle $P_{max}$, d.h. F(WB) ist zu niedrig.

4.2.4 Berechnung eines neuen Vollblutflusses

$$F'(WB) = F(WB) + \Delta F(WB)$$

4.2.5 Parameterkopplung an den neuen Wert F'(WB) nach 2.2 bis 2.5

4.2.6 falls $P_{ein} > P_{max}$, weiter mit 4.2.4

4.2.7 falls $P_{ein} < P_{max}$, weiter mit 4.l.2 (d.h. Flußoptimierungsphase beendet)

4.3 Ständige Vollblutflußoptimierung

Bei dieser Variante wird für die Dauer der ganzen Behandlung der maximal mögliche Vollblutfluß geregelt.

(Anm.: Ist $P_{min} = P_{max}$ und $\Delta F(WB)$ sehr klein, dann erfolgt mit einer Regelprozedur nach 4.3 eine Ausregelung eines konstanten Eingangsdruckes)

$$P_{ein}$$

F(WB) zu klein

$$P_{max}$$

zulässiger Bereich

$$P_{min}$$

F(WB) zu groß

Es sind nachstehende Verfahrensschritte vorgesehen:

4.3.l wie 4.l.l

4.3.2 wie 4.l.2

4.3.3 wie 4.2.3

4.3.4 wie 4.2.4

4.3.5 wie 4.2.5

4.3.6 wie 4.2.6

4.3.7 falls $P_{ein} > P_{min}$, weiter mit 4.3.3

4.3.8 falls $P_{ein} < P_{min}$ : $F'(WB) = F(WB) - \Delta F(WB)$

4.3.9 Parameterkopplung an den neuen Wert von F(WB) 2.2 bis 2.5

4.3.l0 wie 4.l.6

4.3.ll weiter mit 4.3.3

(d.h. ständige Flußoptimierung)

Der Mikroprozessor arbeitet somit auch als für die Vollblutzufuhr innerhalb des Steuerzyklus zur optimalen Einstellung der anderen Betriebsparameter.

**Patentansprüche**

1. Verfahren zur Trennung der Bestandteile des einem Spender "in vivo" einstellbar entnommenen Blutes (Vollblut), das einstellbar mit Antikoagulantien versetzt, einer die Trennung bewirkenden, in der Drehzahl einstellbaren Blutzentrifuge zugeführt wird, aus der die separierten Bestandteile (Blutfraktionen) einstellbar abgezogen und danach zum Teil gesammelt sowie zum Teil zum Spender rückgeführt werden, bei dem die Fließraten des Vollblutes und der Fraktionen gemessen und selbsttätig so eingestellt werden, daß eine optimale Trennung erfolgt, dadurch gekennzeichnet, daß man bei einer Änderung des Vollblutflusses die Drehzahl der Zentrifuge in der Weise verändert, daß das Verhältnis der in der Zentrifuge pro Zeiteinheit separierten Volumenteile der Blutfraktionen zum Volumen des in der Zeiteinheit in die Zentrifuge hineingeförderten Vollblutes vor und nach einer Änderung des Vollblutflusses annähernd gleich ist.

2. Verfahren nach Anspruch 1, dadurch gekennzeichnet, daß man bei einer Änderung des Vollblutflusses den Fluß des Antikoagulants in die Zentrifuge hinein und den Fluß jeder Blutfraktion aus der Zentrifuge heraus vor und nach einer Änderung des Vollblutflusses zum Vollblutfluß annähernd im gleichen Verhältnis hält.

3. Verfahren nach Anspruch 1 oder 2, dadurch gekennzeichnet, daß man bei einer Änderung des Vollblutflusses die Drehzahl der Zentrifuge proportional der Quadratwurzel aus dem neu vorgegebenen Vollblutfluß einstellt.

4. Verfahren nach einem der Ansprüche 1-3, dadurch gekennzeichnet, daß die Proportionalitätsfaktoren des Systems vorbestimmt und gespeichert sind.

5. Verfahren nach einem der Ansprüche 1-4, dadurch gekennzeichnet, daß man die Fließrate des Antikoagulants oder der einzelnen Blutfraktionen oder die Drehzahl der Zentrifuge jeweils einzeln, entkoppelt von den anderen Größen, verändert, insbesondere ohne Rückwirkung auf den Vollblutfluß, indem der zugehörige Proportionalitätsfaktor geändert und der geänderte Wert abgespeichert wird.

6. Verfahren nach einem der Ansprüche 1-5, bei dem die Fließrate des Vollblutflusses getrennt geregelt wird, dadurch gekennzeichnet, daß man die Fließrate des Vollblutes - und gekoppelt damit die anderen Systemgrößen - entsprechend dem am Eingang der Blutpumpe pro Zeiteinheit angebotenen Blutvolumen einstellt.

7. Vorrichtung zur Steuerung eines Systems zur Trennung der Bestandteile des einem Spender (1) "in vivo" entnommenen Blutes (Vollblutes) mit mindestens einer Blutzentrifuge (2), der Vorrichtungen (3, 4, 10, 12) zur Blutentnahme, zur Förderung von Antikoagulantien, des entnommenen Blutes in die Blutzentrifuge und der separierten Bestandteile (Blutfraktionen), die zum Teil gesammelt und zum Teil zum Spender rückgeführt werden, zugeordnet sind, mit Steuer-/Regelanordnungen (6, 8, 9, 12, 13) zur Einstellung der Fließraten des Antikoagulant, des Vollblutes sowie der verschiedenen Blutfraktionen und der Drehzahl der Zentrifuge, und mit einem Sensor (16) zur Erfassung der Fließrate des Vollblutes, dessen Signal auf eine Steuerstufe (15) geschaltet ist, deren Ausgang mit den Steuer-/Regelanordnungen verbunden ist, dadurch gekennzeichnet, daß die Übertragungsfunktion der Steuerstufe so bestimmt ist, daß bei einer Änderung des Vollblutflusses die Drehzahl (n) der Zentrifuge in der Weise veränderbar ist, daß das Verhältnis der in der Zentrifuge pro Zeiteinheit separierten Volumenteile der Blutfraktionen ($F_1$, $F_2$) zum Volumen des in der Zeiteinheit in die Zentrifuge hineingeförderten Vollblutes vor und nach einer Änderung des Vollblutflusses annähernd gleich ist.

8. Vorrichtung nach Anspruch 7, dadurch gekennzeichnet, daß die Steuerstufe (15) zusätzlich mit den Fördervorrichtungen für Antikoagulant und Blutfraktionen verbunden ist und ihre Übertragungsfunktion so bestimmt ist, daß bei einer Änderung des Vollblutflusses der Fluß des Antikoagulants in die Zentrifuge hinein und der Fluß jeder Blutfraktion aus der Zentrifuge heraus vor und nach einer Änderung des Vollblutflusses zum Vollblutfluß annähernd das gleiche Verhältnis zueinander aufweisen.

9. Vorrichtung nach Anspruch 7 oder 8, dadurch gekennzeichnet, daß die Übertragungsfunktion der Steuerstufe (15) so bestimmt ist, daß bei einer Änderung des Vollblutflusses und des zugehörigen Eingangssignals am Eingang der Steuerstufe das Ausgangssignal zur Einstellung der Drehzahl der Zentrifuge (2) proportional der Quadratwurzel aus dem neu eingestellten Vollblutfluß ist.

10. Vorrichtung nach Anspruch 9, dadurch gekennzeichnet, daß die Steuerstufe (15) zusätzlich mit den Fördervorrichtungen (4, 10, 11) für Antikoagulant und Blutfraktionen verbunden ist und ihre Übertragungsfunktion so bestimmt ist, daß bei einer Änderung des Vollblutflusses zusätzlich Ausgangssignale für die Einstellung der Fließraten von Antikoagulantien und aller Blutfraktionen erzeugbar sind, die proportional der Größe des Vollblutflusses sind.

11. Vorrichtung nach Anspruch 9 und 10, dadurch gekennzeichnet, daß die Proportionalitätsfaktoren (Koppelkonstanten $c_4$, $c_5$, $c_n$, mit n = Index der Fraktion) des Systems vorbestimmt und in Form von digitalen oder analogen Größen in der Steuerstufe gespeichert sind.

12. Vorrichtung nach Anspruch 11, dadurch gekennzeichnet, daß die Steuerung so getroffen ist, daß die Fließrate des Antikoagulants oder der einzelnen Blutfraktionen oder die Drehzahl der Zentrifuge jeweils einzeln, entkoppelt von den anderen Größen, veränderbar sind, insb. ohne Rückwirkung auf den Vollblutfluß, indem die zugehörige Koppelkonstante geändert wird und der geänderte Wert in der Steuerstufe abgespeichert ist.

13. Vorrichtung nach einem der Ansprüche 7 bis 12, dadurch gekennzeichnet, daß die Steuerstufe (15) ein Mikroprozessorsystem ist.

14. Vorrichtung nach einem der Ansprüche 7 bis 13, dadurch gekennzeichnet, daß die Vorrichtungen (4, 7, 10, 11) zur Förderung der Antikoagulantien, des Vollblutes und der Blutfraktionen peristaltisch arbeitende Schlauchpumpen sind, denen jeweils eine Steuer- oder Regeleinrichtung (6, 8, 12, 13) für die Drehzahl zugeordnet ist, über die der Durchsatz (Fließrate) einstellbar ist.

15. Vorrichtung nach einem der Ansprüche 7 bis 14, mit einem der Fördervorrichtung für das Vollblut (Blutpumpe) unterlagerten Regelsystem für eine Änderung der Fließrate des Vollblutes, dadurch gekennzeichnet, daß das Regelsystem (8) so aufgebaut ist, daß es die Fließrate des Vollblutes - und gekoppelt damit die anderen Systemgrößen - so einstellt, wie es dem am Eingang der Blutpumpe (7) pro Zeiteinheit angebotenen Blutvolumen entspricht.

16. Vorrichtung nach Anspruch 15, dadurch gekennzeichnet, daß ein Drucksensor (16) für den Eingangsdruck der Blutpumpe vorgesehen ist, der das Istwertsignal für das Regelsystem (8) bildet.

17. Vorrichtung nach Anspruch l6, dadurch gekennzeichnet, daß der Sollwert ein vorbestimmter Druckwert ist, so daß das Regelsystem den Vollblutfluß der Blutpumpe so einstellt, daß der Eingangsdruck einen konstanten, dem Sollwert entsprechenden Wert annimmt.

18. Steuerung nach Anspruch 16, dadurch gekennzeichnet, daß dem Regelsystem ein vorbestimmter Sollwertbereich zugeordnet ist, so daß der Fluß der Blutpumpe so einstellbar ist, daß der Eingangsdruck in dem Sollwertbereich liegt.

**Claims**

1. Method of separating the constituents of the blood (full blood) which is taken adjustably from a donor "in vivo", adjustable mixed with anticoagulants, supplied to a speed-adjustable blood centrifuge effecting the separation and from which the separated constituents (blood fractions) are adjustably withdrawn and thereafter partly collected and partly returned to the donor, wherein the flow rates of the full blood and of the fractions are measured and automatically adjusted so that an optimum separation takes place, characterized in that on a variation of the full blood flow the speed of rotation of the centrifuge is varied in such a manner that the ratio of the volume parts of the blood fractions separated in the centrifuge per unit time to the volume of the full blood introduced into the centrifuge in the unit time is approximately the same ratio before and after a change in the full blood flow.

2. Method according to claim 1, characterized in that on a change in the full blood flow the flow of the anticoagulant into the centrifuge and the flow of each blood fraction out of the centrifuge have approximately the same ratio to the full blood flow before and after a change in the full blood flow.

3. Method according to claim 1 or 2, characterized in that on a change in the full blood flow the speed of rotation of the centrifuge is set proportionally to the square root of the newly set full blood flow.

4. Method according to any one of claims 1 to 3, characterized in that the proportionality factors of the system are predetermined and stored.

5. Method according to any one of claims 1 to 4, characterized in that the flow rate of the anticoagulant or the individual blood fractions or the speed of rotation of the centrifuge are each individually variable decoupled from the other parameters, in particular without reaction on the full blood flow, in that the associated proportionality factor is changed and the changed value is stored.

6. Method according to any one of claims 1 to 5 wherein the flow rate of the full blood flow is separately regulated, characterized in that the flow rate of the full blood - and coupled thereto the other system parameters - is set so that it corresponds to the blood volume offered per unit time at the inlet of the blood pump.

7. Apparatus for controlling a system for separating the constituents of the blood (full blood) taken from a donor (1) "in vivo" comprising at least one blood centrifuge (2) with which means (3, 4, 10, 12) are associated for blood withdrawal, delivery of anticoagulants, of the removed blood to the blood centrifuge and of the separated constituents (blood fractions) which are partially collected and partially returned to the donor, control/regulating means (6, 8, 9, 12, 13) for adjusting the flow rates of the anticoagulant, the full blood and the various blood fractions and the speed of rotation of the centrifuge, and a sensor (16) for detecting the flow rate of the full blood, the signal of which is switched to a control stage (15) whose output is connected to the control/regulating means, characterized in that the transfer function of the control stage is so defined that on a change in the full blood flow the speed of rotation (n) of the centrifuge is variable in such a manner that the ratio of the volume parts of the blood fractions ($F_1$, $F_2$) separated in the centrifuge per unit time to the volume of the full blood conveyed in the unit time into the centrifuge is approximately the same ratio before and after a change in the full blood flow.

8. Apparatus according to claim 7, characterized in that the control stage (15) is additionally connected to the delivery means for anticoagulant and blood fractions and its transfer function is so defined that on a change in the full blood flow the flow of the anticoagulant into the centrifuge and the flow of each blood fraction out of the centrifuge has approximately the same ratio to the full blood flow before and after a change in the full blood flow.

9. Apparatus according to claim 7 or 8, characterized in that the transfer function of the control stage (15) is so defined that on a change in the full blood flow and the associated input signal at the input of the control stage the output signal for setting the speed of rotation of the centrifuge (10) is proportional to the square root of the newly set full blood flow.

10. Apparatus according to claim 9, characterized in that the control stage (15) is additionally connected to the delivery means (4, 10, 11) for anticoagulant and blood fractions and its transfer function is so defined that on a change in the full blood flow additionally output signals can be generated for setting the flow rates of anticoagulants and all the blood fractions which are proportional to the magnitude of the full blood flow.

11. Apparatus according to claim 9 and 10, characterized in that the proportionality factors (coupling constants $c_4$, $c_5$, $c_n$, with n = index of the fraction) of the system are predetermined and stored in the form of digital or analog quantities in the control stage.

12. Apparatus according to claim 11, characterized in that the control is so designed that the flow rate of the anticoagulant or the individual blood fractions or the speed of rotation of the centrifuge are each individually variable, decoupled from the other parameters, in particular without reaction on the full blood flow, in that the associated coupling constant is changed and the changed value is stored in the control stage.

13. Apparatus according to any one of claims 7 to 12, characterized in that the control stage (15) is a microprocessor system.

14. Apparatus according to any one of claims 7 to 13, characterized in that the means (4, 7, 10, 11) for delivering the anticoagulants, the full blood and the blood fractions are peristaltically operating hose pumps with each of which a control or regulating means (6, 8, 12, 13) is associated for the speed of rotation via which the throughput (flow rate) is adjustable.

15. Apparatus according to any one of claims 7 to 14 comprising a control system based on the delivery means for the full blood (blood pump) for changing the flow rate of the full blood, characterized in that the control system (8) is so designed that it adjusts the flow rate of the full blood - and coupled thereto the other system parameters - in such a manner that it corresponds to the blood volume offered per unit time at the inlet of the blood pump (7).

**16.** Apparatus according to claim 15, characterized in that a pressure sensor (16) is provided for the inlet pressure of the blood pump and forms the actual value signal for the control system (8).

**17.** Apparatus according to claim 16, characterized in that the desired value is a predetermined pressure value so that the control system sets the full blood flow of the blood pump such that the inlet pressure assumes a constant value corresponding to the desired value.

**18.** Control according to claim 16, characterized in that associated with the control system is a predetermined desired value range such that the flow of the blood pump is adjustable so that the inlet pressure lies in the desired value range.

**Revendications**

**1.** Procédé pour la séparation des constituants du sang (sang complet) prélevé de manière réglable "in vivo" sur un donneur, le sang étant mélangé de manière réglable à des anticoagulants, envoyé vers une centrifugeuse à sang, de vitesse réglable, opérant la séparation, d'où les constituants séparés (fractions sanguines) sont extraits de manière réglable puis en partie recueillis et en partie renvoyés au donneur, dans lequel procédé les taux d'écoulement du sang complet et des fractions sont mesurés et automatiquement réglés de manière qu'il se produise une séparation optimale, caractérisé en ce que lors d'une variation du flux de sang complet, la vitesse de rotation de la centrifugeuse est modifiée de manière que le rapport entre les parties de volume des fractions sanguines, séparées par unité de temps dans la centrifugeuse, et le volume du sang complet envoyé par unité de temps dans la centrifugeuse, soit à peu près le même avant et après une variation du flux de sang complet.

**2.** Procédé selon la revendication 1, caractérisé en ce qu'en cas de variation du flux de sang complet, le flux d'anticoagulant envoyé dans la centrifugeuse et le flux de chaque fraction sanguine sortant de la centrifugeuse est maintenu à peu près dans le même rapport vis-à-vis du flux de sang complet, avant et après une variation de ce flux.

**3.** Procédé selon la revendication 1 ou 2, caractérisé en ce que dans le cas d'une variation du flux de sang complet, la vitesse de rotation de la centrifugeuse s'établit proportionnellement à la racine carrée du nouveau flux de sang complet.

**4.** Procédé selon l'une des revendications 1 à 3, caractérisé en ce que les facteurs de proportionnalité du système sont prédéterminés et mis en mémoire.

**5.** Procédé selon l'une des revendications 1 à 4, caractérisé en ce que les taux d'écoulement de l'anticoagulant ou des diverses fractions sanguines ou la vitesse de la centrifugeuse sont modifiés individuellement, indépendamment des autres grandeurs, notamment sans rétroaction sur le flux de sang complet, en ce que le facteur de proportionnalité correspondant est modifié et la valeur modifiée mise en mémoire.

**6.** Procédé selon l'une des revendications 1 à 5 dans lequel les taux d'écoulement du flux de sang complet sont réglés séparément, caractérisé en ce que les taux d'écoulement du sang complet - et en liaison avec ceux-ci les autres grandeurs du système - sont réglés en fonction du volume de sang se présentant par unité de temps à l'entrée de la pompe à sang.

**7.** Dispositif destiné à la commande d'un système de séparation des constituants du sang (sang complet) prélevé "in vivo" sur un donneur (1) avec au moins une centrifugeuse à sang (2) à laquelle sont associés des dispositifs (3, 4, 10, 12) destinés au prélèvement du sang, à l'envoi d'anticoagulants, du sang prélevé vers la centrifugeuse à sang et des constituants séparés (fractions sanguines) qui sont en partie recueillis et en partie renvoyés au donneur, avec des dispositifs de commande/régulation (6, 8, 9, 12, 13) destinés à régler les taux d'écoulement de l'anticoagulant, du sang complet ainsi que des différentes fractions sanguines et la vitesse de rotation de la centrifugeuse et avec un capteur (16) destiné à enregistrer les taux d'écoulement du sang complet, dont le signal est envoyé à un étage de commande (15) dont la sortie est reliée aux dispositifs de commande/régulation, caractérisé en ce que la fonction de transmission de l'étage de commande est telle qu'en cas de variation du flux de sang complet, la vitesse (n) de la centrifugeuse peut être modifiée de manière que le rapport entre les parties de volume des fractions sanguines ($F_1$, $F_2$), séparées par unité de temps dans la centrifugeuse, et le volume du sang complet envoyé par unité de temps dans la centrifugeuse, soit à peu près le même avant et après variation du flux de sang complet.

8. Procédé selon la revendication 7, caractérisé en ce que l'étage de commande (15) est en outre relié aux dispositifs de transport de l'anticoagulant et des fractions sanguines et en ce que sa fonction de transmission est telle qu'en cas de variation du flux de sang complet, le flux de l'anticoagulant envoyé dans la centrifugeuse et le flux de chaque fraction sanguine sortant de la centrifugeuse présentent à peu près le même rapport vis-à-vis du flux de sang complet, avant et après variation du flux de sang complet.

9. Dispositif selon la revendication 7 ou 8, caractérisé en ce que la fonction de transmission de l'étage de commande (15) est telle qu'en cas de variation du flux de sang complet et du signal d'entrée correspondant, à l'entrée de l'étage de commande, le signal de sortie destiné a régler la vitesse de rotation de la centrifugeuse (2) est proportionnel à la racine carrée du nouveau flux de sang complet.

10. Dispositif selon la revendication 9, caractérisé en ce que l'étage de commande (15) est relié en outre aux dispositifs de transport (4, 10, 11) de l'anticoagulant et des fractions sanguines et sa fonction de transmission est telle qu'en cas de variation du flux de sang complet, des signaux de sortie destinés à régler les taux d'écoulement des anticoagulants et de toutes les fractions sanguines peuvent en outre être engendrés, proportionnellement à la valeur du flux de sang complet.

11. Dispositif selon les revendications 9 et 10, caractérisé en ce que les facteurs de proportionnalité (constantes de couplage $c_4$, $c_5$, $c_n$ avec n = indice de la fraction) du système sont prédéterminés et mis en mémoire dans l'étage de commande, sous la forme de grandeurs numériques ou analogiques.

12. Dispositif selon la revendication 11, caractérisé en ce que la commande est telle que les taux d'écoulement de l'anticoagulant ou des différentes fractions sanguines ou la vitesse de rotation de la centrifugeuse peuvent être modifiés individuellement, indépendamment des autres grandeurs, notamment sans rétroaction sur le flux de sang complet, en ce que la constante de couplage correspondante est modifiée et la valeur modifiée mise en mémoire dans l'étage de commande.

13. Dispositif selon l'une des revendications 7 à 12, caractérisé en ce que l'étage de commande (15) est un système à microprocesseur.

14. Dispositif selon l'une des revendications 7 à 13, caractérisé en ce que les dispositifs (4, 7, 10, 11) destinés au transport des anticoagulants, du sang complet et des fractions sanguines sont des pompes tubulaires péristaltiques à chacune desquelles est associé un dispositif de commande ou de régulation (6, 8, 12, 13) pour la vitesse de rotation, par lesquelles le débit (taux d'écoulement) peut être réglé.

15. Dispositif selon l'une des revendications 7 a 14, avec un système de régulation asservi au dispositif de transport du sang complet (pompe à sang) pour une variation des taux d'écoulement du sang complet, caractérisé en ce que le système de régulation (8) est conçu de telle manière que les taux d'écoulement du sang complet-et couplées avec ceux-ci les autres grandeurs du système-soient réglés en fonction du volume de sang se présentant par unité de temps à l'entrée de la pompe à sang (7).

16. Dispositif selon la revendication 15, caractérisé en ce qu'il est prévu un capteur de pression (16) pour la pression d'entrée de la pompe à sang, lequel forme le signal de valeur réelle pour le système de régulation (8).

17. Dispositif selon la revendication 16, caractérisé en ce que la valeur de consigne est une valeur de pression prédéterminée, de sorte que le système de régulation établit le flux de sang complet de la pompe à sang de manière que la pression d'entrée prenne une valeur constante, correspondant à la valeur de consigne.

**18.** Dispositif selon la revendication 16, caractérisé en ce qu'un domaine de valeur de consigne prédéterminé est associé au système de régulation, de manière que le flux de la pompe à sang soit réglable de telle sorte que la pression d'entrée se situe dans le domaine de valeur de consigne.

Fig. 1

Fig. 2

EP 0 261 530 B1

Fig. 3